# EUROPÄISCHE PATENTSCHRIFT

(11) **EP 3 488 759 B1**
(45) Veröffentlichungstag und Bekanntmachung des Hinweises auf die Patenterteilung: **01.04.2020**
(21) Anmeldenummer: 18207217.3
(22) Anmeldetag: 20.11.2018
(51) Int. Cl.: A61B 1/00

(54) **OPTISCHES INSTRUMENT**
OPTICAL INSTRUMENT
INSTRUMENT OPTIQUE

(30) Priorität: 24.11.2017 DE 102017127827
(43) Veröffentlichungstag der Anmeldung: 29.05.2019
(73) Patentinhaber: Karl Storz SE & Co. KG, 78532 Tuttlingen (DE)
(72) Erfinder: Maichle, Stefanie, 78532 Tuttlingen (DE); Egle, Michael, 78532 Tuttlingen (DE); Kramer, Claus, 78532 Tuttlingen (DE)
(74) Vertreter: Hofmeister, Frank

(56) Entgegenhaltungen:
- EP-A1- 1 031 054
- EP-A1- 3 225 149
- US-B1- 6 246 823

## Beschreibung

Die Erfindung betrifft ein optisches Instrument für die minimal invasive Chirurgie mit einem Instrumentengehäuse zur Aufnahme optischer Elemente, wobei das Instrumentengehäuse distalseitig und/oder proximalseitig über mindestens ein Endfenster fluiddicht verschlossen ist, das in einer das Endfenster vollumfänglich umschließenden Fensterfassung angeordnet ist.

Endoskope und andere medizinische optische Instrumente für die minimal invasive Chirurgie müssen vor jedem Einsatz sterilisiert werden. Diese Sterilisation erfolgt heutzutage mittels Autoklavierung, wobei die optischen Instrumente einer Behandlung mit Heißdampf bei über 3 bar Druck und einer Temperatur von über 130° C ausgesetzt werden.

Ein gattungsgemäßes optisches Instrument für die minimal invasive Chirurgie ist beispielsweise aus der DE 195 07 205 C2 bekannt.

Um sicherzustellen, dass auch bei der hohen thermischen Belastung des Autoklavierens keine Feuchtigkeit in das Instrumentengehäuse eindringt, ist es aus der Praxis bekannt, die als Deckgläser und/oder Endlinsen verwendeten endseitigen Endfenster durch Schweißen oder Löten fluiddicht mit dem Instrumentengehäuse zu verbinden.

Zum Einlöten der Endfenster in die entsprechende Fensterfassung des Instrumentengehäuses weisen die Endfenster eine metallische Randbeschichtung auf, wodurch sie sich mit Zinn- oder Goldlot in die dafür vorgesehene Fensterfassung einlöten lassen. Beim Einlöten in die Fensterfassung ist das jeweilige Endfenster von einem flüssigen Lotbad umgeben. In dieser Lage, in der das Endfenster quasi in dem Lotbad schwimmt, ist es schwierig das Endfenster exakt mittig in der Fensterfassung anzuordnen. Eine außermittig Lage des Endfensters führt dann automatisch zu einer unterschiedlich dicken Lotschicht zwischen dem Endfenster und der Fensterfassung.

Aufgrund unterschiedlicher thermischer Ausdehnungskoeffizienten der verwendeten Materialien des Instrumentengehäuses, der Fensterfassung und des Lots kommt es insbesondere bei der außermittigen Platzierung der Endfenster in der Fensterfassung immer wieder zu erheblichen Spannungen, die zu Rissbildungen und/oder Spannungsbrüchen an den Endfenstern und somit zu Undichtigkeiten führen können.

Davon ausgehend liegt der Erfindung die **Aufgabe** zugrunde, ein optisches Instrument für die minimal invasive Chirurgie zu schaffen, bei dem die Endfenster spannungsarm in der zugehörigen Fensterfassung festlegbar sind.

Die **Lösung** dieser Aufgabenstellung ist erfindungsgemäß dadurch gekennzeichnet, dass an der dem jeweiligen Endfenster zugewandten inneren Umfangsfläche einer jeden Fensterfassung mindestens zwei von der Fensterfassung radial nach innen ragende Abschnitte ausgebildet sind.

Durch die Ausbildung einzelner separater von der Fensterfassung radial nach innen ragende Abschnitte wird das jeweilige Endfenster während des Einlötens mittig in der Fensterfassung gehalten, da aufgrund der radial nach innen ragenden Abschnitte kaum Platz für das Endfenster für eine außermittige Verlagerung verbleibt. Diese Ausbildung ermöglicht somit, dass sich über den gesamten zu verlötenden Umfang des Endfensters ein im Wesentlichen gleichmäßig dicker Lotrand ausbilden kann. Durch diesen gleichmäßig dicken Lotrand um das jeweilige Endfenster lässt sich die beim Löten und Abkühlen auftretende Spannung auf das jeweilige Endfenster deutlich reduzieren, so dass Spannungsrisse oder Spannungsbrüche an den Endfenstern nicht mehr auftreten.

Mit einer bevorzugten Ausführungsform der Erfindung wird vorgeschlagen, dass an jeder Fensterfassung drei oder vier radial nach innen ragende Abschnitte ausgebildet sind.

Die Ausbildung von drei oder vier radial nach innen ragenden Abschnitten hat sich als besonders effektiv herausgestellt, um eine mittige Platzierung des Endfensters in der jeweiligen Fensterfassung zu gewährleisten und andererseits ausreichend Freiraum zur Ausbildung des im Wesentlichen gleichmäßig dicken Lotrandes bereitzustellen.

Selbstverständlich ist es erfindungsgemäß auch möglich, mehr als vier radial nach innen ragende Abschnitte an der zugehörigen Fensterfassung vorzusehen.

Weiterhin wird mit der Erfindung vorgeschlagen, dass zwischen den radialen Innenflächen der radial nach innen ragenden Abschnitte einer jeden Fensterfassung und dem zugehörigen Endfenster ein Spalt zur Ausbildung einer Lotschicht ausgebildet ist. Dieser verbleibende Spalt zwischen der Fensterfassung und dem Endfenster gewährleistet die Ausbildung des das Endfenster vollumfänglich umgebenden Lotrandes, der die fluiddichte Abdichtung gewährleistet.

Zur Ausbildung der radial nach innen ragende Abschnitte wird erfindungsgemäß vorgeschlagen, dass jeder von der Fensterfassung radial nach innen ragende Abschnitt als wellenförmige Vorwölbung ausgebildet ist.

Aufgrund der wellenförmigen Vorwölbung ergibt sich eine quasi nur punktuelle Ausbildung des Bereichs, in dem der Lotrand zwischen der Fensterfassung und dem Endfenster besonders dünn ausgebildet ist.

Die wellenförmigen Ausbildung der radial nach innen ragenden Abschnitte der Fensterfassung ist einerseits ausreichend, um das Endfenster mittig in der Fensterfassung zu platzieren und gewährleistet andererseits die Ausbildung eines im Wesentlichen gleichmäßig dicken Lotrands, wodurch die Ausbildung möglicher Spannungen aufgrund unterschiedlicher thermischer Ausdehnungskoeffizienten der verwendeten Materialien erheblich reduziert wird.

Um eine mittige Lage der Endfenster in der zugehörigen Fensterfassung zu gewährleisten, wird mit der Erfindung vorgeschlagen, dass alle wellenförmigen Vorwölbungen einer Fensterfassung den gleichen Wölbungsradius aufweisen. Selbstverständlich ist es möglich, dass bei einem optischen Instrument mit mehreren Endfenstern die Wölbungsradien der wellenförmigen Vorwölbungen einer jeden Fensterfassung sich von den Wölbungsradien der anderen Fensterfassungen des gleichen optischen Instruments unterscheiden.

Das Auftreten möglicher Spannungen aufgrund unterschiedlicher thermischer Ausdehnungskoeffizienten der verwendeten Materialien kann erfindungsgemäß weiterhin dadurch reduziert werden, dass die radial nach innen ragenden Abschnitte gleichmäßig über den inneren Umfang der jeweiligen Fensterfassung verteilt angeordnet sind. Dies bedeutet, dass, beispielsweise bei der Verwendung von drei Abschnitten diese um 120° und bei vier Abschnitten diese um 90°, versetzt zueinander über den inneren Umfang der Fensterfassung verteilt angeordnet sind.

Zur Ausbildung der Fensterfassungen zur Aufnahme der Endfenster wird mit der Erfindung vorgeschlagen, dass das distale und/oder proximale Ende des Instrumentengehäuses die Fensterfassung bildet. Diese direkte Ausbildung der distalen und/oder proximalen Gehäuseinnenwand als Fensterfassung kommt insbesondere bei optischen Instrumenten mit einem kleinen Schaftdurchmesser, beispielsweise kleiner 10mm, zur Anwendung.

Schließlich wird mit einer alternativen Ausführungsform der Erfindung vorgeschlagen, dass die Fensterfassung als am distalen und/oder proximalen Ende des Instrumentengehäuses festlegbares separates Bauteil ausgebildet ist. Diese Ausbildung Fensterfassung als separates Bauteil, das an der distalen und/oder proximalen Gehäuseinnenwand festlegbar ist, kommt insbesondere bei optischen Instrumenten mit einem größeren Schaftdurchmesser, beispielsweise 10mm und größer, zur Anwendung.

Weitere Merkmale und Vorteile der Erfindung ergeben sich anhand der zugehörigen Zeichnungen, in denen ein Ausführungsbeispiel eines erfindungsgemäßen optischen Instruments für die minimal invasive Chirurgie nur beispielhaft dargestellt ist, ohne die Erfindung auf dieses Ausführungsbeispiel zu beschränken. In den Zeichnungen zeigt:
- Fig. 1: einen schematischen Längsschnitt durch ein als Endoskop ausgebildetes optisches Instruments für die minimal invasive Chirurgie gemäß dem Stand der Technik;
- Fig. 2: eine geschnittene Vorderansicht des distalen Endes eines erfindungsgemäßen optischen Instruments und
- Fig. 3: eine vergrößerte Darstellung des Details III gemäß Fig. 2.

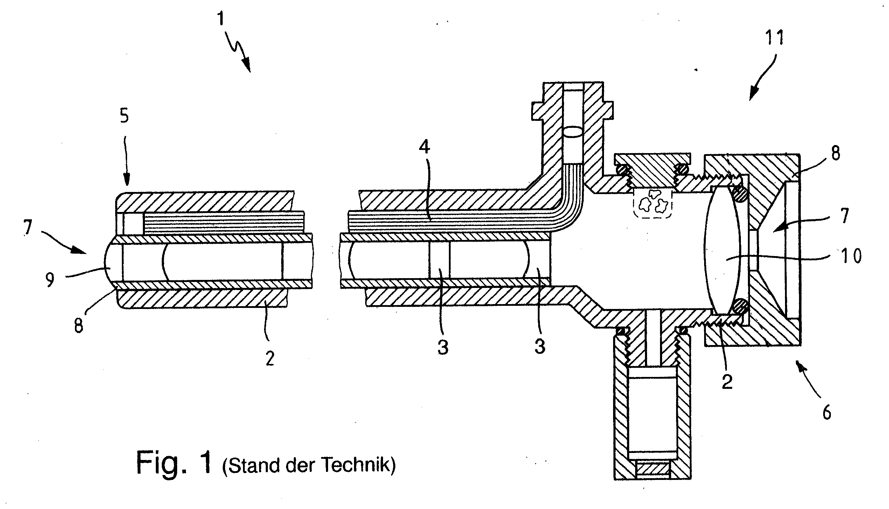

Die Abbildung Fig. 1 zeigt schematisch den Aufbau eines als Endoskop ausgebildeten optischen Instruments 1 für die minimal invasive Chirurgie. Das optische Instrument 1 weist ein hohles Instrumentengehäuse 2 auf, in dem verschiedene optische Elemente, wie beispielsweise Linsen 3 und ein Lichtleiterfaserbündel 4 angeordnet sind.

Am distalen Ende 5 sowie am proximalen Ende 6 ist das Instrumentengehäuse 2 über jeweils ein Endfenster 7 fluiddicht verschlossen, das in einer das Endfenster 7 vollumfänglich umschließenden Fensterfassung 8 angeordnet ist.

Bei dem in Fig. 1 dargestellten optischen Instrument 1 ist das distalseitige Endfenster 7 als Endlinse 9 und das proximalseitige Endfenster 7 als Deckglas 10 in einer Okulareinheit 11 ausgebildet.

Endoskope und andere medizinische optische Instrumente 1 müssen vor jedem Einsatz sterilisiert werden. Diese Sterilisation erfolgt heutzutage mittels Autoklavierung, wobei die optischen Instrumente 1 einer Behandlung mit Heißdampf bei über 3 bar Druck und einer Temperatur von über 130° C ausgesetzt werden.

Um sicherzustellen, dass auch bei der hohen thermischen Belastung des Autoklavierens keine Feuchtigkeit in das Instrumentengehäuse 2 eindringt, werden die als Deckgläser 10 und/oder Endlinsen 9 verwendeten endseitigen Endfenster 7 insbesondere durch thermische Fügeverfahren, wie Schweißen oder Löten, fluiddicht mit dem Instrumentengehäuse 2 verbunden. In aller Regel sind Klebeverfahren zum Einfügen der Endfenster 7 nicht geeignet, da die Klebverbindungen den thermischen Belastungen des Autoklavierens auf Dauer nicht standhalten.

Beim Autoklavieren der optischen Instrumente 1 können aufgrund unterschiedlicher thermischer Ausdehnungskoeffizienten der verwendeten Materialien bei mit dem Instrumentengehäuse 2 verschweißten oder verlöteten Endfenstern 7 erhebliche Spannungen auftreten, die zu Rissbildungen und/oder Spannungsbrüchen der Endfenster 7 und somit zu Undichtigkeiten führen können.

Diese Spannungen können bei optischen Instrumenten 1 gemäß dem Stand der Technik dadurch auftreten, dass beim Einlöten in die Fensterfassung 8 das jeweilige Endfenster 7 von einem flüssigen Lotbad umgeben und nur mit einem Lötdorn fixiert ist. Zum Einlöten der Endfenster 7 in die entsprechende Fensterfassung 8 des Instrumentengehäuses 2 weisen die Endfenster 7 eine metallische Randbeschichtung auf, wodurch sie sich mit Zinn- oder Goldlot in die dafür vorgesehene Fensterfassung 8 einlöten lassen.

In dieser Lage, in der das Endfenster 7 quasi in dem Lotbad schwimmt, ist es insbesondere bei abgewinkelten Optiken schwierig, das Endfenster 7 exakt mittig in der Fensterfassung 8 anzuordnen. Eine außermittig Lage des Endfensters 7 führt dann automatisch zu einer unterschiedlich dicken Lotschicht zwischen dem Endfenster 7 und der Fensterfassung 8, so dass unterschiedliche Wärmedehnungen beim Löten und Wärmestauchungen beim nachfolgenden Abkühlen auftreten können.

Zur Ausbildung der Fensterfassungen 8 zur Aufnahme der Endfenster 7 stehen prinzipiell zwei verschiedene Ausführungsformen zur Verfügung.

Vorzugsweise bei optischen Instrumenten 1 mit einem kleinen Schaftdurchmesser des Instrumentengehäuses 2, beispielsweise einem Durchmesser von kleiner 10mm, bildet insbesondere am distalen Ende 5 des Instrumentengehäuses 2 die Gehäuseinnenwand des Instrumentengehäuses 2 direkt die Fensterfassung 8.

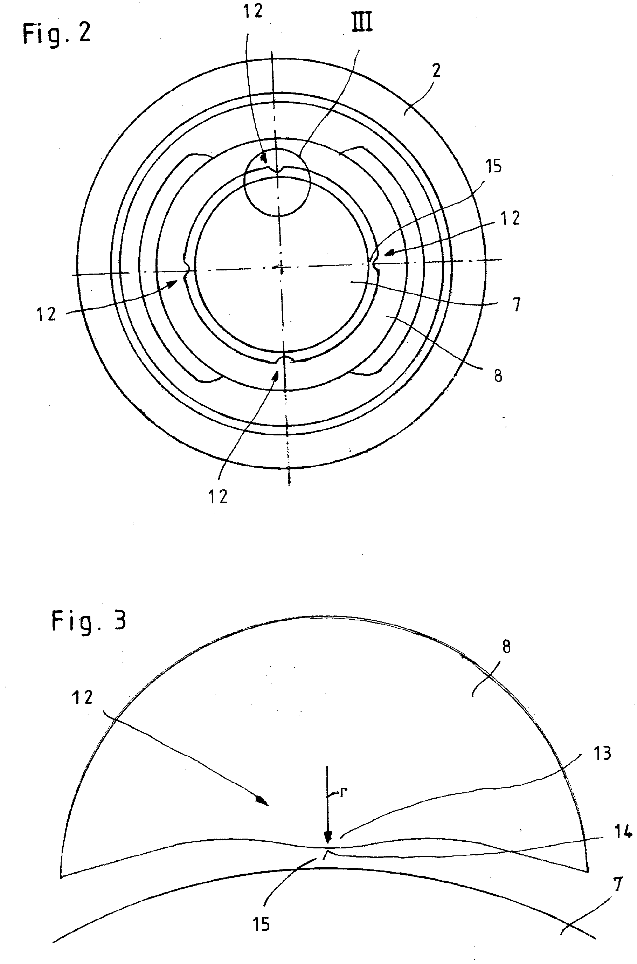

Bei optischen Instrumenten 1 mit einem größeren Schaftdurchmesser des Instrumentengehäuses 2, beispielsweise 10mm und größer, ist insbesondere am distalen Ende 5 des Instrumentengehäuses 2 die Fensterfassung 8 als separates Bauteil ausgebildet, das an der distalen Gehäuseinnenwand des Instrumentengehäuses 2 festlegbar ist, wie dies in Fig. 2 dargestellt ist.

Der Aufbau der Fensterfassung 8 wird nachstehend anhand der Abbildungen Fig. 2 und 3 erläutert.

Um ein optisches Instrument 1 zu schaffen, bei dem die Endfenster 7 spannungsarm in der zugehörigen Fensterfassung 8 festlegbar sind, sind an der dem jeweiligen Endfenster 7 zugewandten inneren Umfangsfläche einer jeden Fensterfassung 8 mindestens zwei von der Fensterfassung 8 radial nach innen ragende Abschnitte 12 ausgebildet.

Wie insbesondere aus Fig. 3 ersichtlich, sind die radial nach innen ragende Abschnitte 12 vorzugsweise als wellenförmige Vorwölbungen 13 der Fensterfassung 8 ausgebildet. Die radiale Höhe der nach innen ragende Abschnitte 12 ist so bemessen, dass zwischen den radialen Innenflächen 14 der radial nach innen ragenden Abschnitte 12 einer jeden Fensterfassung 8 und dem zugehörigen Endfenster 7 ein Spalt 15 zur Ausbildung einer Lotschicht verbleibt.

Durch die Ausbildung einzelner separater von der Fensterfassung 8 radial nach innen ragende Abschnitte 12 wird das jeweilige Endfenster 7 während des Einlötens mittig in der Fensterfassung 8 gehalten, da aufgrund der radial nach innen ragenden Abschnitte 12 und des nur geringen Spalts 15 zwischen den radialen Innenflächen 14 der Abschnitte 12 und dem Außenumfang des Endfensters 7 kaum Platz für eine außermittige Verlagerung des Endfensters 7 verbleibt. Diese Ausbildung ermöglicht somit, dass sich über den gesamten zu verlötenden Umfang des Endfensters 8 ein im Wesentlichen gleichmäßig dicker Lotrand ausbilden kann. Durch diesen gleichmäßig dicken Lotrand um das jeweilige Endfenster 7 lässt sich die beim Löten und Abkühlen auftretende Spannung auf das jeweilige Endfenster 7 deutlich reduzieren, so dass Spannungsrisse oder Spannungsbrüche an den Endfenstern 7 nicht mehr auftreten.

Bei der in Fig. 2 dargestellten Ausführungsform weist die Fensterfassung vier als wellenförmige Vorwölbungen 13 ausgebildete radial nach innen ragende Abschnitte 12 auf. Selbstverständlich ist es auch möglich, nur drei oder mehr als vier radial nach innen ragende Abschnitte 12 an jeder Fensterfassung 8 vorzusehen.

In der Abbildung Fig. 2 sind die wellenförmigen Vorwölbungen 13 in den Proportionen zur Fensterfassung 8 und zum Endfenster 7 vergrößert und nicht maßstabsgetreu dargestellt, um die Ausbildung der radial nach innen ragenden Abschnitte 12 anschaulich darstellen zu können.

Wie weiterhin aus Fig. 2 ersichtlich, sind die vier radial nach innen ragenden Abschnitte 12 um 90° versetzt zueinander gleichmäßig über den inneren Umfang der Fensterfassung 8 verteilt angeordnet. Die gleichmäßige Verteilung der radial nach innen ragenden Abschnitte 12 über den inneren Umfang der Fensterfassung 8, die mit entsprechenden Winkelabständen auch bei einer anderen Anzahl an radial nach innen ragenden Abschnitte 12 vorteilhaft ist, verringert das Auftreten von Spannungen, da so die Ausbildung des Lotrandes über den Umfang des Endfensters 7 weiterhin vergleichmäßigt wird.

Um eine mittige Lage der Endfenster 7 in der zugehörigen Fensterfassung 8 zu gewährleisten, weisen alle wellenförmigen Vorwölbungen 13 einer Fensterfassung 8 den gleichen Wölbungsradius r auf.

Selbstverständlich ist es möglich, dass bei einem optischen Instrument 1 mit mehreren Endfenstern 7 die Wölbungsradien r der wellenförmigen Vorwölbungen 13 einer jeden Fensterfassung 8 sich von den Wölbungsradien r der anderen Fensterfassungen 8 des gleichen optischen Instruments 1 unterscheiden.

Eine wie zuvor beschrieben ausgestaltete Fensterfassung 8 eines optischen Instruments 1 zeichnet sich somit dadurch aus, dass aufgrund der Ausbildung der von der Fensterfassung 8 radial nach innen ragenden Abschnitte 12 das in die Fensterfassung 8 einzulötende Endfenster 7 immer mittig in der Fensterfassung 8 platzierbar ist. Dies gewährleistet gleichzeitig die Ausbildung eines im Wesentlichen gleichmäßig dicken Lotrands zwischen der Fensterfassung 8 und dem Endfenster 7, wodurch beim Löten und Abkühlen auftretende Spannung auf das jeweilige Endfenster 7 deutlich reduziert werden können, so dass Spannungsrisse oder Spannungsbrüche an den Endfenstern 7 nicht mehr auftreten.

### Bezugszeichenliste

- 1: optisches Instrument
- 2: Instrumentengehäuse
- 3: Linse
- 4: Lichtleitfaserbündel
- 5: distales Ende
- 6: proximales Ende
- 7: Endfenster
- 8: Fensterfassung
- 9: Endlinse
- 10: Deckglas
- 11: Okulareinheit
- 12: Abschnitt
- 13: Vorwölbung
- 14: Innenfläche
- 15: Spalt

- r: Wölbungsradius

## Patentansprüche

1. Optisches Instrument für die minimal invasive Chirurgie mit einem Instrumentengehäuse (2) zur Aufnahme optischer Elemente, wobei das Instrumentengehäuse (2) distalseitig und/oder proximalseitig über mindestens ein Endfenster (7) fluiddicht verschlossen ist, das in einer das Endfenster (7) vollumfänglich umschließenden Fensterfassung (8) angeordnet ist,
**dadurch gekennzeichnet,**
**dass** an der dem jeweiligen Endfenster (7) zugewandten inneren Umfangsfläche einer jeden Fensterfassung (8) mindestens zwei von der Fensterfassung (8) radial nach innen ragende Abschnitte (12) ausgebildet sind.

2. Optisches Instrument nach Anspruch 1, **dadurch gekennzeichnet, dass** an jeder Fensterfassung (8) drei oder vier radial nach innen ragende Abschnitte (12) ausgebildet sind.

3. Optisches Instrument nach Anspruch 1 oder 2, **dadurch gekennzeichnet, dass** jeder von der Fensterfassung (8) radial nach innen ragende Abschnitt (12) als wellenförmige Vorwölbung (13) ausgebildet ist.

4. Optisches Instrument nach Anspruch 3, **dadurch gekennzeichnet, dass** alle wellenförmigen Vorwölbungen (13) einer Fensterfassung (8) den gleichen Wölbungsradius (r) aufweisen.

5. Optisches Instrument nach einem der Ansprüche 1 bis 4, **dadurch gekennzeichnet, dass** die von der Fensterfassung (8) radial nach innen ragenden Abschnitte (12) gleichmäßig über den inneren Umfang der jeweiligen Fensterfassung (8) verteilt angeordnet sind.

6. Optisches Instrument nach einem der Ansprüche 1 bis 5, **dadurch gekennzeichnet, dass** zwischen den radialen Innenflächen (14) der radial nach innen ragenden Abschnitte (12) einer jeden Fensterfassung (8) und dem zugehörigen Endfenster (7) ein Spalt (15) zur Ausbildung einer Lotschicht ausgebildet ist.

7. Optisches Instrument nach einem der Ansprüche 1 bis 6, **dadurch gekennzeichnet, dass** das distale Ende (5) und/oder proximale Ende (6) des Instrumentengehäuses (2) die Fensterfassung (8) bildet.

8. Optisches Instrument nach einem der Ansprüche 1 bis 6, **dadurch gekennzeichnet, dass** die Fensterfassung (8) als am distalen Ende (5) und/oder proximalen Ende (6) des Instrumentengehäuses (2) festlegbares separates Bauteil ausgebildet ist.

## Claims

1. Optical instrument for minimally invasive surgery, having an instrument housing (2) for housing optical elements, wherein the instrument housing (2) is closed in a fluid-tight manner on the distal side and/or on the proximal side by way of at least one end window (7), which is arranged in a window mount (8) that entirely encloses the end window (7),
**characterized**
**in that** at least two portions (12) projecting radially inwardly from the window mount (8) are formed on the inner circumferential face, facing the respective end window (7), of each window mount (8).

2. Optical instrument according to Claim 1,
**characterized in that** three or four radially inwardly projecting portions (12) are formed on each window mount (8).

3. Optical instrument according to Claim 1 or 2,
**characterized in that** each portion (12) projecting radially inwardly from the window mount (8) is embodied in the form of a wave-shaped protrusion (13).

4. Optical instrument according to Claim 3,
**characterized in that** all wave-shaped protrusions (13) of a window mount (8) have the same radius of curvature (r) .

5. Optical instrument according to one of Claims 1 to 4, **characterized in that** the portions (12) projecting radially inwardly from the window mount (8) are distributed uniformly over the inner circumference of the respective window mount (8).

6. Optical instrument according to one of Claims 1 to 5, **characterized in that** a gap (15) for forming a solder layer is formed between the radial inner faces (14) of the radially inwardly projecting portions (12) of each window mount (8) and the associated end window (7) .

7. Optical instrument according to one of Claims 1 to 6, **characterized in that** the distal end (5) and/or proximal end (6) of the instrument housing (2) forms the window mount (8).

8. Optical instrument according to one of Claims 1 to 6, **characterized in that** the window mount (8) is embodied in the form of a separate component that is fixable to the distal end (5) and/or proximal end (6) of the instrument housing (2).

## Revendications

1. Instrument optique pour la chirurgie invasive minimale avec un boîtier d'instruments (2) destiné à loger des éléments optiques, dans lequel le boîtier d'instruments (2) est fermé de manière étanche au fluide côté distal et/ou côté proximal par l'intermédiaire d'au moins une fenêtre d'extrémité (7), qui est disposée dans un cadre de fenêtre (8) entourant la fenêtre d'extrémité (7) sur toute sa périphérie,
**caractérisé en ce**
**qu'**au moins deux parties (12) faisant saillie radialement vers l'intérieur du cadre de fenêtre (8) sont réalisées sur la surface périphérique intérieure, tournée vers la fenêtre d'extrémité (7) respective, de chaque cadre de fenêtre (8).

2. Instrument optique selon la revendication 1, **caractérisé en ce que** trois ou quatre parties (12) faisant saillie radialement vers l'intérieur sont réalisées sur chaque cadre de fenêtre (8).

3. Instrument optique selon la revendication 1 ou 2, **caractérisé en ce que** chaque partie (12) faisant saillie radialement vers l'intérieur du cadre de fenêtre (8) est réalisée sous la forme d'une pré-courbure ondulée (13).

4. Instrument optique selon la revendication 3, **caractérisé en ce que** toutes les pré-courbures ondulées (13) d'un cadre de fenêtre (8) présentent le même rayon de courbure (r).

5. Instrument optique selon l'une quelconque des revendications 1 à 4, **caractérisé en ce que** les parties (12) faisant saillie radialement vers l'intérieur du cadre de fenêtre (8) sont disposées de manière répartie uniformément sur la périphérie intérieure du cadre de fenêtre (8) respectif.

6. Instrument optique selon l'une quelconque des revendications 1 à 5, **caractérisé en ce qu'**une fente (15) pour la réalisation d'une couche de brasure est réalisée entre les surfaces intérieures radiales (14) des parties (12) faisant saillie radialement vers l'intérieur de chaque cadre de fenêtre (8) et la fenêtre d'extrémité (7) associée.

7. Instrument optique selon l'une quelconque des revendications 1 à 6, **caractérisé en ce que** l'extrémité distale (5) et/ou l'extrémité proximale (6) du boîtier d'instruments (2) forment le cadre de fenêtre (8).

8. Instrument optique selon l'une quelconque des revendications 1 à 6, **caractérisé en ce que** le cadre de fenêtre (8) est réalisé sous la forme d'une pièce séparée pouvant être fixée à l'extrémité distale (5) et/ou l'extrémité proximale (6) du boîtier d'instrument (2).
